# EUROPEAN PATENT SPECIFICATION

(11) **EP 3 326 683 B1**
(45) Date of publication and mention of the grant of the patent: **15.03.2023**
(21) Application number: 16827689.7
(22) Date of filing: 13.07.2016
(51) Int. Cl.: A61M 37/00

(54) **PERCUTANEOUS ADMINISTRATION DEVICE**
VORRICHTUNG FÜR PERKUTANE VERABREICHUNG
DISPOSITIF D'ADMINISTRATION PERCUTANÉE

(30) Priority: 17.07.2015 JP 2015143508
(43) Date of publication of application: 30.05.2018
(73) Proprietor: Toppan Printing Co., Ltd., Tokyo 110-0016 (JP)
(72) Inventor: UENO, Hisami, Tokyo 110-0016 (JP)
(74) Representative: TBK
(86) International application number: PCT/JP2016/070699
(87) International publication number: WO 2017/014129

(56) References cited:
- WO-A1-2014/041531
- CN-A- 101 912 663
- JP-A- 2008 154 849
- JP-A- 2010 063 666
- JP-A- 2011 142 968
- US-A1- 2004 260 251
- US-A1- 2013 165 872

## Description

### [Technical Field]

The present invention relates to transdermal administration devices used for drug administration.

### [Background Art]

Transdermal administration devices include an administration section for administering a drug through the skin of a drug administration target. A microneedle, which is an example of the administration section, includes a plurality of needle-shaped projections and a plate-shaped substrate. The plurality of projections are arranged on the surface of the substrate. In a drug administration method using the microneedle, the substrate is first pressed against the skin of the administration target to pierce the skin using the projections. Then, a drug is introduced into the body of the administration target through the holes created by the projections. Such a microneedle is formed of, for example, a water-soluble resin or a biocompatible resin such as a biodegradable resin (for example, see PTLs WO 2006/080508 A1 and JP 2005-21677 A).

WO2014041531 describes an array of microneedles having extensions going through the substrate.

JP 2010063666 describes a microneedle substrate structure having projections extending from one side of the substrate and beams extending from the opposite surface to provide strength.

### [Summary of the Invention]

### [Technical Problem]

The invention is defined in the appended claims 1-9.

A site of the skin surface to be punctured by the projections is predetermined depending on how the effect of the drug is achieved. The site is typically a curved surface that gradually curves in one or more specific directions. Further, in order to accurate delivery of the drug into the body of the administration target, the respective projections need to be inserted into the administration target by an intended depth. For that purpose, it is required that the microneedle is positioned along the surface of the skin, that is, the substrate of the microneedle is bent conforming to the curved surface described above.

Meanwhile, each time the transdermal administration device is used, the direction of the external force applied on the substrate by a user of the transdermal administration device varies to a certain extent even if it is used by the same user or for the same site. Since the flexural rigidity of the substrate is typically substantially the same across the surface of the substrate, the substrate will be bent in any direction in accordance with the external force applied by a user. As a result, even if it is used by the same user or for the same site, the shape of the substrate varies to a certain extent each time the transdermal administration device is used, and accordingly, conformability of the substrate to the skin surface also varies.

Further, a transdermal administration device according to the preamble of claim 1 is known from US 2004/260251 A1. Other transdermal administration devices are disclosed in CN 101 912 663 A.

The present invention has an object of providing a transdermal administration device that reduces variation in the shape of the substrate which occurs when an external force is applied on the substrate of the administration section.

### [Solution to Problem]

A transdermal administration device for solving the above problem includes: an administration section which includes a substrate having a first surface and a second surface which is a surface opposite to the first surface, and a projection protruding from the first surface, the administration section having flexibility. Further, the transdermal administration device includes a support body having a plate shape and a support surface that supports the second surface, the support body having flexibility. The support surface is a flat surface and is bonded to the second surface by adhesiveness of an adhesive applied. The support body includes at least one low rigidity region and at least one high rigidity region having flexural rigidity higher than flexural rigidity of the low rigidity region, the support body being configured to satisfy at least one of requirements that the low rigidity region is sandwiched by two high rigidity regions to form a stripe and that the high rigidity region is sandwiched by two low rigidity regions to form a stripe.

According to the above configuration, a bendable portion of the support body is likely to be formed at the low rigidity region and is not likely to be formed at the high rigidity region for bending the support body by an external force applied. Accordingly, even if the direction of the external force applied on the substrate varies each time the external force is applied, the support body is readily bent along with the substrate at the bendable portion, which is the low rigidity region, and is not readily bent at the high rigidity region. As a result, when an external force is applied on the substrate, the shape of the substrate is guided by the stripes to become either a shape that is hardly bent at positions of the stripes or a shape that is largely bent at positions of the stripes. Accordingly, variation in the shape of the substrate can be reduced.

Further, in the transdermal administration device according to the present invention, the support body has a raised and/or recessed surface which is a surface opposite to the support surface, the high rigidity region includes a raised element raised from the raised and/or recessed surface, and the low rigidity region includes a recessed element recessed from the raised and/or recessed surface.

According to the above configuration, the high rigidity region and the low rigidity region can be easily defined in the support body since the distribution of rigidity on the support body can be determined by a level difference on the raised and/or recessed surface.

In the above transdermal administration device, the stripe may be formed of a single structure that extends in a strip shape.

According to the above configuration, since a single structure having a strip shape forms the stripe, reproducibility of the shape of the substrate which is guided by the stripes can be improved.

In the above transdermal administration device, the stripe may be composed of a plurality of intermittent structures.

According to the above configuration, since the stripe is composed of a plurality of intermittent structures, a more complex shape of the substrate which is guided by the stripes may be easily contemplated.

The above transdermal administration device may include a plurality of the stripes, and the plurality of stripes may include the stripes that do not intersect with each other.

The above transdermal administration device may include a plurality of the stripes, and the plurality of stripes may include the stripes having an end and disposed adjacent to each other, and connected to each other at the ends.

According to these configurations, a portion of the support body which is easily bent or a portion of the support body which is not easily bent is prevented from being complexly branched. Accordingly, these configurations are suitable for the application of a substrate which is to be largely bent compared with the configuration having complex branches of these portions.

The above transdermal administration device may include a plurality of the stripes, and the plurality of stripes may include stripes that extend in different directions from each other.

The size of the curved surface or the curvature of the curved surface of the skin of the administration target is slightly different for each administration site to which the transdermal administration device is applied. With the above configuration having stripes that extend in different directions, the support body can be more easily deformed along the shape of the administration target, and thus the substrate can be more easily bent along the administration target.

The above transdermal administration device may include a plurality of stripes, and the plurality of stripes may include at least one stripe that extends in a straight line.

According to the above configuration, the shape guided by the stripes can be simplified, and the arrangement of the stripes can be easily designed.

The above transdermal administration device may include a plurality of stripes, and the plurality of stripes may include at least one stripe that extends in a curved line.

According to the above configuration, the shape guided by the stripes can be more complex.

In the above transdermal administration device, the support body may have an adhesive surface outside the substrate as viewed in the direction perpendicular to the first surface.

According to the above configuration, the substrate can be easily bent along the administration target by the adhesive surface adhered to the administration target. Then, the transdermal administration device can be fixed and retained to the administration target when having an optimal shape for administration. Further, when the adhesive surface has high adhesiveness so that the transdermal administration device is fixed to the administration target with the substrate disposed along the administration target, the projections can remain pierced into the administration target for a sufficient period of time.

### [Advantageous Effects of Invention]

According to the present invention, variation in the shape of the substrate which occurs when an external force is applied on the substrate of the administration section can be reduced.

### [Brief Description of the Drawings]

Fig. 1 is a perspective view which illustrates a perspective structure of a transdermal administration device of an embodiment.
Fig. 2 is a plan view which illustrates a plan structure of a transdermal administration device of an embodiment.
Fig. 3 is a cross-sectional view which illustrates a cross-sectional structure of a transdermal administration device of an embodiment.
Fig. 4 is a plan view which illustrates another example of a plan structure of a transdermal administration device of an embodiment.
Fig. 5 is a plan view which illustrates another example of a plan structure of a transdermal administration device of an embodiment.
Fig. 6 is a plan view which illustrates another example of a plan structure of a transdermal administration device of an embodiment.
Fig. 7 is a plan view which illustrates another example of a plan structure of a transdermal administration device of an embodiment.
Fig. 8 is a plan view which illustrates another example of a plan structure of a transdermal administration device of an embodiment.
Fig. 9 is a plan view which illustrates another example of a plan structure of a transdermal administration device of an embodiment.
Fig. 10 is a cross-sectional view which illustrates another example of a cross-sectional structure of a transdermal administration device of an embodiment.
Fig. 11 is a cross-sectional view which illustrates a cross-sectional structure of a transdermal administration device of a first modified example.
Fig. 12 is a cross-sectional view which illustrates a cross-sectional structure of a transdermal administration device of a second modified example.
Fig. 13 is a cross-sectional view which illustrates a cross-sectional structure of a transdermal administration device of a third modified example.
Fig. 14 is a cross-sectional view which illustrates a cross-sectional structure of a transdermal administration device of a fourth modified example.
Fig. 15 is a cross-sectional view which illustrates a cross-sectional structure of a transdermal administration device of a fifth modified example.
Fig. 16 is a cross-sectional view which illustrates a cross-sectional structure of a transdermal administration device of a sixth modified example.
Fig. 17 is a cross-sectional view which illustrates a cross-sectional structure of a transdermal administration device of a seventh modified example.

### [Description of Embodiments]

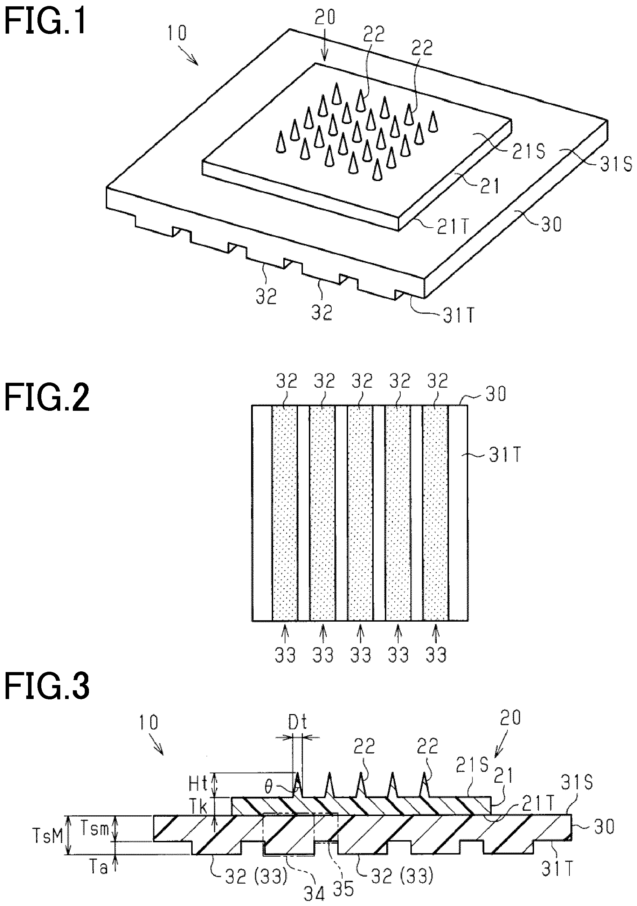

With reference to Figs. 1 to 10, an embodiment of a transdermal administration device will be described.

### [Configuration of Transdermal Administration Device]

As shown in Fig. 1, a transdermal administration device 10 includes a microneedle 20 which is an example of an administration section, and a support substrate 30 which is an example of a support body that supports the microneedle 20.

The microneedle 20 includes a plate shaped substrate 21 having flexibility and a plurality of projections 22 which protrudes from a first surface 21S of the substrate 21. The substrate 21 has the first surface 21S on which the projections 22 are disposed and a second surface 21T which is a surface opposite to the first surface 21S. The first surface 21S supports the proximal ends of the projections 22.

The outer shape of the substrate 21 as viewed in the direction perpendicular to the first surface 21S is not specifically limited, and may be a rectangle, circle, or oval.

The projection 22 may be a cone or pyramid shape. Further, the projection 22 may be a shape which does not have a pointed tip, for example, a cylinder or prism shape. Further, the projection 22 may be a shape composed of a combination of two or more three dimensional shapes, for example, a cone stacked on a cylinder. In other words, the projection 22 may be any shape that can pierce the skin. Moreover, the projection 22 may have a narrow portion or shoulder on the side wall thereof.

The number of projections 22 is not specifically limited, but is one or more. When the microneedle 20 includes a plurality of projections 22, the plurality of projections 22 may be arranged regularly or irregularly as viewed in the direction perpendicular to the first surface 21S of the substrate 21. For example, the plurality of projections 22 is arranged in a matrix or concentric pattern.

The support substrate 30 is located on the surface of the substrate 21 of the microneedle 20 opposite to the projections 22 and supports the substrate 21. Specifically, the support substrate 30 has a flexible plate shape, and has a facing surface 31S which is an example of a support surface that supports the second surface 21T of the substrate 21, and a raised and/or recessed surface 31T which is a surface opposite to the facing surface 31S. The facing surface 31S, which is a flat surface, is oriented to the second surface 21T of the substrate 21 and is bonded to the second surface 21T by adhesiveness of an adhesive applied. The raised and/or recessed surface 31T includes raised and/or recessed elements 32 disposed on a strip, and each of the raised and/or recessed elements 32 is either a raised element raised from the raised and/or recessed surface 31T or a recessed element recessed from the raised and/or recessed surface 31T. Fig. 1 illustrates an example where the raised and/or recessed elements 32 are composed of the raised elements.

The outer shape of the support substrate 30 as viewed in the direction perpendicular to the first surface 21S is not specifically limited, but the outer edge of the support substrate 30 is preferably aligned with the outer edge of the substrate 21 or located outside the outer edge of the substrate 21. In other words, the entire substrate 21 is overlapped with the support substrate 30 when viewed in the direction perpendicular to the first surface 21S. Moreover, the entire substrate 21 is preferably overlapped with the region in which the raised and/or recessed elements 32 are disposed as viewed in the direction perpendicular to the first surface 21S.

Fig. 2 is a diagram of the transdermal administration device 10 as viewed in the direction perpendicular to the raised and/or recessed surface 31T of the support substrate 30, and the raised elements on the raised and/or recessed surface 31T are indicated by dot hatching.

As shown in Fig. 2, each of the raised and/or recessed elements 32 has a strip shape extending along the surface of the facing surface 31S, forming a stripe 33 with a strip-like outer shape. In the example shown in Fig. 2, the raised and/or recessed surface 31T includes a plurality of raised and/or recessed elements 32. Each of the raised and/or recessed elements 32 is a single raised element, has a strip shape extending in one direction, and forms one stripe 33. The raised and/or recessed element 32 has a constant width in the direction perpendicular to the extending direction of the raised and/or recessed element 32. Each of the plurality of raised and/or recessed elements 32 form the individual stripes 33, and as a result of that, a plurality of stripes 33 is formed by the plurality of raised and/or recessed elements 32.

The adjacent raised and/or recessed elements 32 are spaced from each other and extend in the same direction. That is, the plurality of stripes 33 extend in a straight line in the same direction and do not intersect with each other. Each of the plurality of stripes 33 connects the opposing ends of the raised and/or recessed surface 3 1T. In other words, each of the plurality of stripes 33 extends from one side of the rectangular raised and/or recessed surface 31T to the opposing side.

With reference to Fig. 3, dimensions of the projections 22 and the raised and/or recessed elements 32 will be described in detail.

As shown in Fig. 3, the projection 22 has a length Ht which is a maximum length of the projection 22 in a thickness direction of the substrate 21, that is, a direction perpendicular to the surface of the first surface 21S of the substrate 21, which is a length from the first surface 21S of the substrate 21 to the tip of the projection 22. The length Ht of the projection 22 is determined depending on the purpose of the puncture to be created by the projection 22, the type of the drug to be administered, and the depth of the puncture hole required to be created by the projection 22. Preferably, the length Ht of the projection 22 is in the range of 20 µm or more and 1000 µm or less.

When the puncture target is the human skin and the depth of the puncture hole created by the projection 22 is set to be a depth that passes through the stratum corneum and does not reach the nerve fibers, the length Ht of the projection 22 is preferably in the range of 200 µm or more and 700 µm or less, and more preferably in the range of 200 µm or more and 600 µm or less.

When the depth of the hole is in such a range that penetrates the stratum corneum and does not reach nerve fibers, the drug can be delivered to a site deeper than the stratum corneum. Since the hole formed in the stratum corneum closes as time elapses, the stratum corneum serves as a barrier to the outside so that the drug delivered deeper than the stratum corneum is held in the body. Accordingly, the drug can be held in the body for a long period of time since the drug is prevented from being peeled off due to the metabolism of the stratum corneum or washing skin for skin care or the like.

Moreover, when the depth of the hole is in such a range that locates it within the stratum corneum, the length Ht of the projection 22 is preferably in the range of 30 µm or more and 300 µm or less, and more preferably in the range of 30 µm or more and 250 µm or less.

Since the depth of the hole is in a range that is in the stratum corneum, the drug can be retained in the stratum corneum. The drug in the stratum corneum is excreted as the time elapses since the stratum corneum is constantly newly produced by metabolism. Accordingly, a state of the drug being retained in the body is easily released, for example, by washing the skin or peeling the skin.

When the microneedle 20 includes the plurality of projections 22, the length Ht of the plurality of projections 22 may be the same or different from each other. For example, in the case where the length Ht of the projections 22 located on the outer periphery area among the plurality of projections 22 is larger than the length Ht of the projections 22 located in the center area, the projections 22 can be easily in contact with a curved surface of the skin when the skin of the administration target is a curved surface. Alternatively, for example, in the case where the length Ht of the projections 22 located on the outer periphery area among the plurality of projections 22 is smaller than the length Ht of the projections 22 located in the center area, the projections 22 located on the outer periphery area, which are susceptible to an external force, may have an improved mechanical strength.

The projection 22 has a width Dt, which is a maximum width of the projection 22 in a direction parallel with the surface of the first surface 21S of the substrate 21. The width Dt of the projection 22 is determined depending on the required aspect ratio of the projection 22 or the required volume of the hole. The width Dt of the projection 22 is preferably in the range of 1 µm or more and 300 µm or less. For example, when the projection 22 has a cone or circular columnar shape, the width Dt of the projection 22 is a diameter of a circle which is the bottom of the projection 22, that is, the bottom which is in contact with the first surface 21S of the substrate 21. Further, for example, when the projection 22 has a regular quadrangular pyramid or regular quadrangular prism shape, the width Dt of the projection 22 is a length of a diagonal of the square which is the bottom of the projection 22 which is in contact with the first surface 21S of the substrate 21.

When the tip of the projection 22 is formed in a pointed shape and the puncture hole is created to penetrate the stratum corneum, the tip angle θ of the projection 22 is preferably in a range of 5° or more and 45° or less, and more preferably in a range of 8° or more and 25° or less.

The tip angle θ of the projection 22 is a maximum angle made by the tip of the projection 22 in a cross section in the thickness direction of the substrate 21. For example, when the projection 22 has a regular quadrangular pyramid shape, the tip angle θ of the projection 22 is an apex angle of a triangle which has a diagonal of a square of the bottom of the projection 22 as a base and the apex of the regular quadrangular pyramid projection 22 as an apex.

Further, the shape of the projection 22 is not limited to the above described shapes, and may be appropriately determined depending on the purpose of puncture to be created by the projection 22 or the type of the drug to be administered. For example, the purpose of the puncture created by the projection 22 may be promotion of percutaneous absorption of the drug or extraction of a substance in the body to the outside the body through the skin. Further, the shape of the projection 22 can be determined in view of improvement in piercing performance to the skin. When the microneedle 20 includes a plurality of projections 22, the plurality of projections 22 may have different shapes from each other.

The substrate 21 has a thickness Tk which is a thickness of the substrate 21 in the thickness direction of the substrate 21 from the first surface 21S to the second surface 21T. The thickness Tk of the substrate 21 is not specifically limited, and is preferably in the range of 10 µm or more and 2 mm or less.

Further, since the microneedle 20 is used to administer a drug via the skin, materials for forming the microneedle 20 are preferably biocompatible materials as described later. The biocompatible materials are expensive compared with general, non-biocompatible materials. Accordingly, the smaller amount of material used for forming the microneedle 20 is more preferred in view of the reduction of manufacturing cost. One strategy for reducing the amount of material used for forming the microneedle 20 is reducing the thickness Tk of the substrate 21. However, the smaller the thickness Tk of the substrate 21, the lower the rigidity of the substrate 21. This leads to variation in the shape of the substrate 21 when an external force is applied on the substrate 21. In particular, this tendency is significant when the rigidity of the substrate 21 is so low that the substrate 21, which has the thickness Tk within the above range, is subject to deformation to an unexpected extent. Therefore, the smaller the thickness Tk of the substrate 21, the greater the advantages of the effect by the transdermal administration device 10 according to the present embodiment, that is, the effect of reducing variation in the shape of the substrate 21 when an external force is applied on the substrate 21. In this sense, when the thickness Tk of the substrate 21 is in the range of 10 µm or more and 500 µm or less, beneficial effects are achieved by the transdermal administration device 10 according to the present embodiment.

Further, when the rigidity of the substrate 21 is so low that the substrate 21 is subject to deformation to an unexpected extent, the larger the area of the surface of the substrate 21 and thus the surface of the first surface 21S, the lower the rigidity of the substrate 21. As a result, the substrate 21 is more likely to be subject to deformation to an unexpected extent. Therefore, the effect brought by the transdermal administration device 10 according to the present embodiment, that is, the effect of reducing variation in the shape of the substrate 21 due to an external force applied on the substrate 21 is more advantageous when the area of the first surface 21S is larger. In this sense, in order to achieve beneficial effects by the transdermal administration device 10 of the present embodiment, the area of the first surface 21S of the substrate 21 is preferably 0.2 cm² or more, more preferably 1 cm² or more, and more preferably 3 cm² or more.

On the other hand, when the rigidity of the substrate 21 is so high that the substrate 21 is not easily bent, the larger the area of the surface of the first surface 21S, the more difficult it is to apply a force to bend the substrate 21 in a specific direction. As a result, the shape of the substrate 21 is more likely to vary when an external force is applied on the substrate 21. Therefore, even if the substrate 21 has high rigidity and is not easily bent, the larger the area of the first surface 21S, the more beneficial effects achieved by the transdermal administration device 10 of the present embodiment.

The area of the first surface 21S is an area of the entire first surface 21S which includes the region in which projections 22 are disposed in the first surface 21S.

The raised elements on the raised and/or recessed surface 31T of the support substrate 30 have a large thickness in the support substrate 30, and are thus located on the surface of the high rigidity region 34 having high flexural rigidity. The recessed elements on the raised and/or recessed surface 31T have a smaller thickness than that of the high rigidity region 34 in the support substrate 30, and are thus located on the surface of the low rigidity region 35 having low flexural rigidity.

A maximum thickness TsM of the support substrate 30 is a maximum thickness of the support substrate 30 in the thickness direction of the substrate 21. That is, the maximum thickness TsM of the support substrate 30 corresponds to a length in the thickness direction of the substrate 21 from the facing surface 31S to the most raised part of the raised and/or recessed surface 31T.

A minimum thickness Tsm of the support substrate 30 is a minimum thickness of the support substrate 30 in the thickness direction of the substrate 21. That is, the minimum thickness Tsm of the support substrate 30 corresponds to a length in the thickness direction of the substrate 21 from the facing surface 31S to the most recessed part of the raised and/or recessed surface 31T.

The maximum thickness TsM of the support substrate 30 preferably has a dimension having rigidity of a degree that would not allow the support substrate 30 to be deformed by its own weight if the support substrate 30 has a constant thickness of the maximum thickness TsM. The maximum thickness TsM is determined depending on the size or material of the support substrate 30. When the support substrate 30 is made of a resin, the maximum thickness TsM is set to be in the range of 50 µm or more and 5 mm or less, for example.

The minimum thickness Tsm of the support substrate 30 preferably has a dimension having rigidity of a degree that would allow the support substrate 30 to be bent when an external force is applied on the support substrate 30 by hand if the support substrate 30 has a constant thickness of the minimum thickness Tsm.

A raised and/or recessed depth Ta corresponds to the amount of level difference on the raised and/or recessed surface 31T in the thickness direction of the substrate 21, which is a length from the top of the raised element to the bottom of the recessed element in the raised element and the recessed element adjacent to each other. The raised and/or recessed depth Ta is determined so that the minimum thickness Tsm falls within a desired range. For example, the raised and/or recessed depth Ta is set to be in the range of 1/20 or more and 19/20 or less of the maximum thickness TsM.

The maximum thickness TsM of the support substrate 30 is preferably larger than the thickness Tk of the substrate 21 in view of easiness of deformation of the substrate 21 conforming to deformation of the support substrate 30. Further, the raised and/or recessed depth Ta is preferably larger than the length Ht of the projection 22 to facilitate guiding the shape of the support substrate 30 by the raised and/or recessed elements 32.

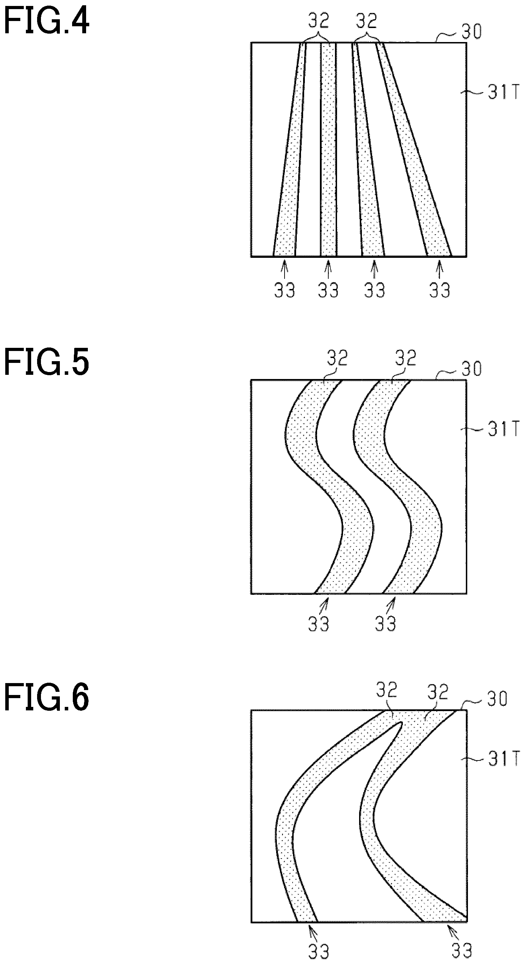

With reference to Figs. 4 to 9, other examples of arrangement of the stripes 33 on the raised and/or recessed surface 31T of the support substrate 30 will be described. Figs. 4 to 9 are diagrams of the transdermal administration device 10 as viewed in the direction perpendicular to the raised and/or recessed surface 31T of the support substrate 30, and the raised elements on the raised and/or recessed surface 31T are indicated by dot hatching.

As shown in Fig. 4, the plurality of stripes 33 may include the stripes 33 that extend in different directions from each other. In the example shown in Fig. 4, each of the raised and/or recessed elements 32 is a single raised element, has a strip shape extending in one direction, and forms one stripe 33. The plurality of raised and/or recessed elements 32 includes the raised and/or recessed elements 32 that extend in different direction from each other, and each of the raised and/or recessed elements 32 form the individual stripes 33. As a result of that, the plurality of stripes 33 which includes the stripes 33 that extend in a straight line in different directions from each other are formed by the plurality of raised and/or recessed elements 32. Each of the plurality of stripes 33 connects the opposing ends of the raised and/or recessed surface 31T.

Preferably, the stripes 33 adjacent to each other do not intersect with each other, or are connected to each other at one of both ends of the stripe 33. In the example shown in Fig. 4, the stripes 33 adjacent to each other do not intersect with each other. Alternatively, in another possible configuration, some of the plurality of stripes 33 are connected to each other at one of both ends of the stripe 33, while the other stripes 33 do not intersect with each other.

The plurality of raised and/or recessed elements 32 may include the raised and/or recessed elements 32 having different shapes from each other as viewed in the direction perpendicular to the raised and/or recessed surface 31T. In the example shown in Fig. 4, the plurality of raised and/or recessed elements 32 includes the raised and/or recessed elements 32 having the width, which is the width in the direction perpendicular to the extending direction of the raised and/or recessed element 32, decreases toward one of both ends of the raised and/or recessed element 32.

As shown in Fig. 5, the stripe 33 may extend in a curved line. In the example shown in Fig. 5, each of the raised and/or recessed elements 32 is a single raised element, has a strip shape extending in a curved line, and forms one stripe 33. Each of the plurality of raised and/or recessed elements 32 have the same curved strip shape, and each of the plurality of stripes 33 form the individual stripes 33. As a result of that, the plurality of stripes 33 extending in a curved line is formed by the plurality of raised and/or recessed elements 32. The plurality of raised and/or recessed elements 32 having the same curved strip shape is arranged in the same orientation. In this configuration, the plurality of stripes 33 extends in the same direction. Each of the plurality of stripes 33 connects the opposing ends of the raised and/or recessed surface 31T.

As shown in Fig. 6, the plurality of stripes 33 that extend in the curved line may include stripes 33 that extend in different directions from each other. In the example shown in Fig. 6, each of the raised and/or recessed elements 32 is a single raised element, has a strip shape extending in a curved line, and forms one stripe 33. The plurality of raised and/or recessed elements 32 includes the raised and/or recessed elements 32 that have a curved strip shape different from each other, and each of the raised and/or recessed elements 32 form the individual stripes 33. As a result of that, the plurality of stripes 33 which includes the stripes 33 that extend in a curved line different from each other is formed by the plurality of raised and/or recessed elements 32. That is, the plurality of stripes 33 includes the stripes 33 that extend in different directions from each other. Each of the plurality of stripes 33 connects the opposing ends of the raised and/or recessed surface 3 1T. Further, the plurality of raised and/or recessed elements 32 having the same curved strip shape may be disposed at different orientations to form the stripes 33 that extend in different directions.

Preferably, the stripes 33 adjacent to each other do not intersect with each other, or are connected to each other at one of both ends of the stripe 33. In the example shown in Fig. 6, the stripes 33 adjacent to each other are connected to each other at one of both ends of the stripe 33. Alternatively, in another possible configuration, some of the plurality of stripes 33 are connected to each other at one of both ends of the stripe 33, while the other stripes 33 do not intersect with each other.

In the examples shown in Figs. 2, and 4 to 6, the recessed elements are formed between the adjacent raised elements of the raised and/or recessed elements 32, and some of the plurality of recessed elements extend in a strip shape on the raised and/or recessed surface 31T. The stripes 33 may be only required to have a shape extending from one side to another side of the raised and/or recessed surface 3 1T. In the example shown in Figs. 2, and 4 to 6, some of the plurality of recessed elements may be regarded as the raised and/or recessed elements 32 that form the stripes 33. In short, in the configuration of the raised and/or recessed surface 31T having both the raised elements raised from the raised and/or recessed surface 31T and the recessed elements recessed from the raised and/or recessed surface 31T, at least one of the requirements that the raised elements each have a strip shape sandwiched by two recessed elements and the recessed elements each have a strip shape sandwiched by two raised elements is satisfied. At least either the raised elements or the recessed elements having a strip shape are the raised and/or recessed elements 32 that constitute the stripes 33. The stripes 33 extend anisotropically, that is, non-uniformly on the raised and/or recessed surface 31T.

Further, in the example shown in Figs. 2, and 4 to 6, the positions of the recessed elements on the raised and/or recessed surface 31T may be modified to those of the raised elements and the positions of the raised elements on the raised and/or recessed surface 31T may be modified to those of the recessed elements to thereby provide a configuration having a reversed pattern of the raised elements and the recessed portions of the raised and/or recessed surface 31T.

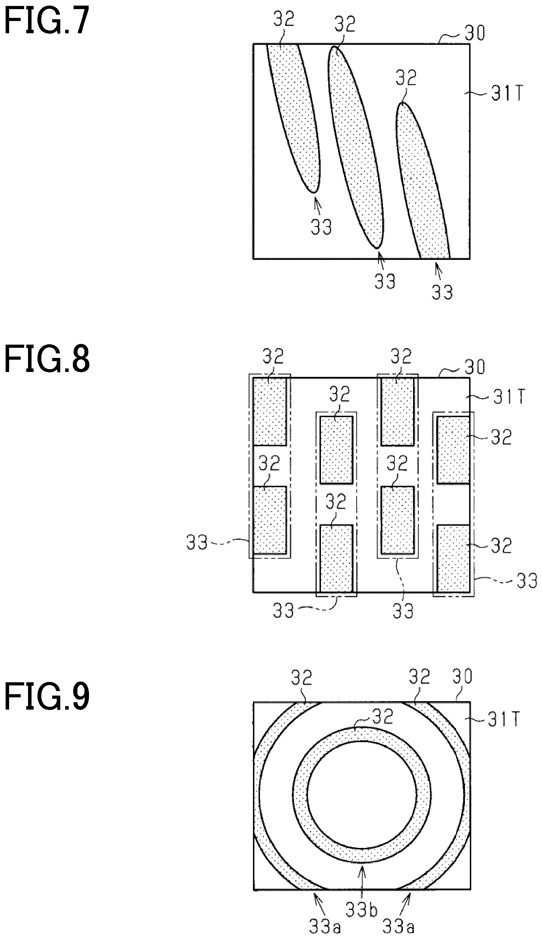

Further, the stripes 33 having a strip shape may not necessarily extend isotropically in all the directions along the facing surface 31S, but only have to extend in specific directions along the facing surface 31S, and may not necessarily connect the opposing ends of the raised and/or recessed surface 31T. For example, in the example shown in Fig. 7, each of the raised and/or recessed elements 32 is a single raised element, has an oval shape or a partial oval shape extending in one direction as viewed in the direction perpendicular to the raised and/or recessed surface 31T, and forms one stripe 33. At least one of both ends of the raised and/or recessed elements 32 in the extending direction thereof does not reach the end of the raised and/or recessed surface 31T.

Although the stripe 33 of the above example is formed of a single structure which is either a raised element or a recessed element that extends in a strip shape, the stripe 33 may be formed of a plurality of structures that are intermittent on a virtual strip. For example, in the example shown in Fig. 8, the stripe 33 is formed of a plurality of structures, which are the raised and/or recessed elements 32. Each of the raised and/or recessed elements 32 is a single raised element having a rectangular shape as viewed in the direction perpendicular to the raised and/or recessed surface 31T. Two raised and/or recessed elements 32 which are intermittent in one direction, in other words, arranged spaced from each other, constitute one stripe 33, and the plurality of stripes 33 are disposed on the raised and/or recessed surface 31T. The plurality of stripes 33 extend in a straight line in the same direction and do not intersect with each other.

The shape of the raised and/or recessed elements 32 that constitute the stripe 33 is not limited to the rectangular shape, and the raised and/or recessed elements 32 may be, for example, a circular shape as viewed in the direction perpendicular to the raised and/or recessed surface 31T.

Further, the raised and/or recessed surface 31T may include the stripes 33 that extend isotropically in all the directions along the facing surface 31S in addition to the stripes 33 that extend anisotropically. For example, in the example shown in Fig. 9, the raised and/or recessed surface 31T includes a stripe 33a and a stripe 33b. The stripe 33a extends in a curved line to connect the opposing ends of the raised and/or recessed surface 3 1T, that is, extends anisotropically on the raised and/or recessed surface 31T. On the other hand, the stripe 33b has an annular shape about the center of the raised and/or recessed surface 31T, that is, extends isotropically on the raised and/or recessed surface 31T. In other words, any configuration is allowable as long as a pattern formed by all the stripes 33 included in the raised and/or recessed surface 31T may extend anisotropically as viewed in the direction perpendicular to the raised and/or recessed surface 31T.

In addition to the aforementioned example, the stripes 33 may have a configuration formed by combining the configurations shown in Figs. 2, and 4 to 9. For example, the stripes 33 shown in Figs. 4 to 7 may be formed of a plurality of raised and/or recessed elements 32 as the configuration shown in Fig. 8. Further, for example, a plurality of stripes 33 may include the stripe 33 that extends in a curved line and the stripe 33 that extends in a straight line.

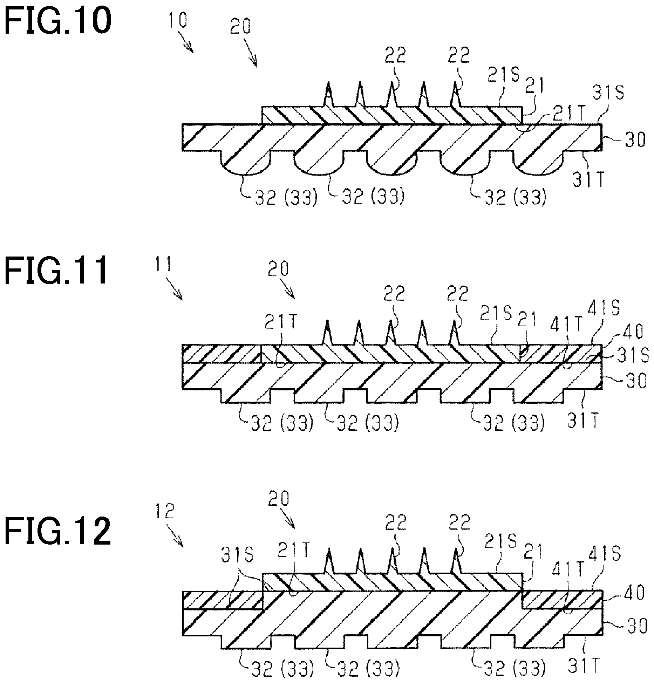

Further, an outer peripheral surface of the raised and/or recessed elements 32 which are the raised elements may be a flat surface as shown in Figs. 1 and 3, or may be a curved surface as shown in Fig. 10. Moreover, an outer peripheral surface of the raised and/or recessed elements 32 which are the raised elements may include both a flat surface and a curved surface. For example, the raised and/or recessed elements 32 which are the raised elements may have a cuboid shape, dome shape, pyramid shape, or cone shape. Similarly, an inner peripheral surface of the raised and/or recessed elements 32 which are the recessed elements may be a flat surface or a curved surface, or an inner peripheral surface of the raised and/or recessed elements 32 may include both a flat surface and a curved surface.

The number of the stripes 33 may be one or more. The number of the stripes 33, the shape of the stripes 33 such as a width and a length of the stripes 33 in the direction along the surface of the first surface 21S of the substrate 21, and the arrangement of the stripes 33 if the plurality of the stripes 33 are provided may be determined depending on the bendability required for the support substrate 30 or a direction in which the support substrate 30 is required to be easily bend.

### [Forming Material and Production Method of Transdermal Administration Device]

The microneedle 20 is preferably made of a biocompatible material. The biocompatible material has little effect on the body, and includes a water soluble polymer, water insoluble polymer, biopolymer, resin, polysaccharides and the like.

The biocompatible material may be a known material. Examples of the biocompatible material include, but are not limited to, carboxymethyl cellulose, methylcellulose, hydroxypropyl cellulose, hydroxypropyl methylcellulose, alginates, curdlan, chitin, chitosan, glucomannan, polymalic acid, collagen, collagen peptide, gelatin, silicon, titanium, silicone, polylactic acid, polyglycolic acid and the like. Further, the saccharides may be trehalose or maltose. Carboxymethyl cellulose, hydroxylpropyl cellulose, or hydroxypropyl methylcellulose may be advantageously used since they have high biological safety. Although there is no clear distinction between chitin and chitosan, chitin with deacetylation of 70% or more is generally referred to as chitosan. Deacetylation may be performed by using a known technique. The chitin, chitosan, or chitosan derivatives having biocompatibility may be substances originating from crustaceans such as crab and shrimp, substances originating from myceliums or microorganism generated plants, or substances using these as starting materials. The chitosan, chitin or chitosan, and chitin or chitosan derivatives are preferable as a forming material of the microneedle 20 since they have an aesthetic effect on the skin and are also sterile and antimicrobial when administered to the skin.

The administration method using the microneedle 20 is not specifically limited. For example, a drug may be applied on the surface of the projections 22 so that the drug is delivered into the skin when the projections 22 pierce the skin. Alternatively, a drug may be contained in the projections 22 so that the drug is delivered into the skin when the projections 22 are dissolved while being pierced into the skin. Further, a liquid drug may be applied on the skin before or after the microneedle 20 is pierced into the skin so that the drug is delivered into the skin through the holes created by the projections 22. Moreover, a drug may be applied by combinations of these techniques. The projections 22 may be removed from the skin or may be embedded into the skin after being pierced into the skin.

The type of the drug is not specifically limited as long as it works when administered into the skin, and may be, for example, physiologically active agents or cosmetic compositions having aesthetic effect. Further, when an aromatic substance is used as a drug, the transdermal administration device 10 suitable for use as a beauty product can be provided since the transdermal administration device 10 generates a fragrance when in use. Moreover, the drug may include biologics. Biologies are drugs which use a raw material or material derived from cells or cell tissues of a human or an animal.

As described above, the drug may be applied on the surface of the projections 22, contained in the projections 22, applied on the skin, or used in the form of a combination thereof depending on the method of drug administration.

The substrate 21 and the projections 22 may be made of a material having the same composition, or materials having different compositions from each other. In the configuration in which the substrate 21 and the projections 22 are made of a material having the same composition, the substrate 21 and the projections 22 can be easily integrally formed.

The microneedle 20 can be produced by using various known techniques. For example, when a resin is used as a material for the microneedle 20, the microneedle 20 can be manufactured by injection molding, extrusion molding, imprinting, hot embossing, casting or the like. Further, the microneedle 20 can be manufactured by machining such as cutting or by etching. Alternatively, an original plate of the microneedle 20 can be formed to produce an intaglio plate having a reversed pattern of raised and recessed portions of the original plate by plating or by molding of a resin and thereby reproduce the microneedle 20 by using the produced intaglio plate.

The support substrate 30 is preferably made of a material that can impart rigidity to the support substrate 30 of such a degree that the support substrate 30 can bear its weight and hold its shape but can be bent when an external force is applied on the support substrate 30 by hand. Specifically, materials for the support substrate 30 include a synthetic resin, paper, wood, and metal.

### [Effects]

Effects of the transdermal administration device 10 of the present embodiment will be described.

Since the support substrate 30 includes the low rigidity region 35 and the high rigidity region 34, a bendable portion of the support substrate 30 is likely to be formed in the low rigidity region of the support body 30 and is not likely to be formed in the high rigidity region 34 for bending the support substrate 30 by an external force applied. The low rigidity region 35 extends along the stripe 33. That is, the low rigidity regions 35 are located adjacent to the stripes 33 when the stripes 33 are composed of raised elements, or the low rigidity regions 35 are located at the stripes 33 when the stripes 33 are composed of recessed elements. As a result, the support substrate 30 is readily bent, for example, along the extending direction of the stripes 33 in the direction perpendicular to the extending direction of the stripes 33. Further, when the extending direction of the stripes 33 changes in middle of the stripes 33, for example, in the case of curved stripes 33, the direction in which the support substrate 30 is readily bent changes accordingly.

Thus, the support substrate 30 is readily bent in a specific direction defined by the stripes 33. Therefore, the substrate 21 of the microneedle 20 supported by the support substrate 30 can be bent in a specific direction by bending the substrate 21 along with the support substrate 30. That is, even if the direction of the external force applied on the substrate 21 varies each time the external force is applied, the support substrate 30 is readily bent along with the substrate 21 at the bendable portion, which is the low rigidity region 35, and is not readily bent at the high rigidity region 34. As a result, when an external force is applied on the substrate 21, the shape of the substrate 21 is guided by the stripes 33 to become either a shape that is hardly bent at positions of the stripes 33 or a shape that is largely bent at positions of the stripes 33.

For example, in the configuration in which the high rigidity regions 34 are located at positions of the stripes 33 and the stripes 33 extend in a straight line, the support substrate 30 is guided to be bent along the extending direction of the stripes 33 with a large radius of curvature when an external force is applied on the support substrate 30. Accordingly, variation in the shape of the substrate 21 can be reduced.

Specifically, when the substrate 21 has low rigidity and is subject to deformation into an unexpected shape, for example, in the case of the substrate 21 made of hydroxypropyl cellulose, a force can be applied on the substrate 21 via the support substrate 30 in the configuration in which the substrate 21 is supported by a support substrate 30 formed to be readily bent into a specific direction. As a result, deformation of the substrate 21 can be easily controlled so that the substrate 21 is bent in a specific direction conforming to deformation of the support substrate 30. Accordingly, variation in the shape of the substrate 21 can be reduced.

Further, when the substrate 21 has high rigidity and is not readily bent, for example, in the case of the substrate 21 made of chitosan, a force can be applied on the substrate 21 via the support substrate 30 formed to be readily bent into a specific direction. As a result, a force for attempting to bend the substrate 21 into the above specific direction is efficiently applied on the substrate 21 compared with the case of attempting to bend only the substrate 21. Accordingly, the substrate 21 is bent into a specific direction conforming to deformation of the support substrate 30, and variation in the shape of the substrate 21 can be reduced.

As described above, the substrate 21 can be readily bent in a specific direction defined by the stripes 33 by bending the substrate 21 of the microneedle 20 along with the support substrate 30. This specific direction can be adapted to the direction in which the skin of the administration target curves so that the substrate 21 can be readily bent along the skin of the administration target when the transdermal administration device 10 is applied to the administration target.

Further, compared with the case of bending the support substrate 30 with a constant maximum thickness TsM, the support substrate 30 is not likely to return to the original shape from the bent state since the support substrate 30 having the stripes 33 is bent at the low rigidity region. Accordingly, the substrate 21 along with the support substrate 30 can remain bent along the skin of the administration target.

Further, in use of the transdermal administration device 10, a user usually holds the transdermal administration device 10 to support the raised and/or recessed surface 31T. Since the raised and/or recessed surface 31T has the raised and/or recessed portions, it is not slippery. Accordingly, the user can easily support the raised and/or recessed surface 31T, and the transdermal administration device 10 can be easily handled. As a result, a decrease in the puncturing function of the projections 22 due to the user unintentionally touching the projection 22 to deform the projection 22 can be prevented.

### [First Modified Example]

A first modified example of the transdermal administration device of the above embodiment will be described.

As shown in Fig. 11, a transdermal administration device 11 of the first modified example includes the microneedle 20, the support substrate 30, and an adhesive sheet 40. The adhesive sheet 40 has an adhesive surface 41S having adhesiveness and a fixation surface 41T which is a surface opposite to the adhesive surface 41S, and is disposed on the facing surface 31S of the support substrate 30 at a position outside the substrate 21 of the microneedle 20. Specifically, the support substrate 30 extends outward from the substrate 21 as viewed in the direction perpendicular to the first surface 21S of the substrate 21, and the fixation surface 41T of the adhesive sheet 40 is fixed to the outwardly extending portion of the substrate 21 on the facing surface 31S of the support substrate 30. The adhesive surface 41S is located outside the substrate 21 as viewed in the direction perpendicular to the first surface 21S of the substrate 21. That is, the support substrate 30 supports the substrate 21 and the adhesive sheet 40.

Further, an outer edge of the adhesive sheet 40 may be located outside or inside the support substrate 30 as viewed in the direction perpendicular to the first surface 21S of the substrate 21, and an outer shape of the adhesive sheet 40 is not limited. Specifically, the configuration in which the outer edge of the adhesive sheet 40 is aligned with the outer edge of the support substrate 30 as viewed in the direction perpendicular to the first surface 21S of the substrate 21 is preferred in view of ease of handling of the transdermal administration device 11. Further, the configuration in which the thickness of the adhesive sheet 40 is approximately the same as or lower than the thickness Tk of the substrate 21 is preferred since the projections 22 can be easily inserted into its proximal end when pierced into the administration target.

For example, the adhesive sheet 40 has a configuration in which an adhesive layer is laminated on both sides of a resin sheet, and a material for forming at least the adhesive surface 41S of the adhesive layer is preferably a biocompatible material. The adhesive surface 41S which is one surface of the adhesive layer preferably has an adhesiveness that can hold the microneedle 20 and retain the adhesive sheet 40 to be adhered to the administration target for a desired period of time.

In use of the transdermal administration device 11, the substrate 21 is bent along with the support substrate 30 and pressed against the skin of the administration target while the adhesive surface 41S of the adhesive sheet 40 is adhered to the skin of the administration target. As a result, the substrate 21 which is bent is disposed along the skin of the administration target, and the microneedle 20 is fixed to the administration target with the projections 22 pierced into the skin.

According to this configuration, the substrate 21 in the transdermal administration device 11 of the adhesive sheet 40 can be easily bent along the skin of the administration target, and the transdermal administration device 11 can be fixed to the administration target while the substrate 21 is disposed along the skin, that is, the shape of the transdermal administration device 11 is optimized for drug administration. As a result, the drug can be properly administered.

### [Second Modified Example]

A second modified example of the transdermal administration device of the above embodiment will be described. The second modified example differs from the first modified example in the configuration of the support substrate 30.

As shown in Fig. 12, in a transdermal administration device 12 of the second modified example, a portion of the support substrate 30 which supports the substrate 21 has a thickness larger than the thickness of the portion which supports the adhesive sheet 40. Accordingly, the first surface 21S of the substrate 21 protrudes from the adhesive surface 41S of the adhesive sheet 40. In the above configuration, the facing surface 31S is a surface having a level difference.

The support substrate 30 can be formed by processing a single sheet to provide a partially different thickness, or laminating a plurality of sheets to form a portion with an increased thickness on the support substrate 30.

According to this configuration, the projections 22 can be easily inserted into the proximal end when pierced into the skin of the administration target since the portion from the distal to the proximal ends of the projection 22 disposed on the first surface 2 1S of the substrate 21 protrudes from the other member of the transdermal administration device 12.

### [Third Modified Example]

A third modified example of the transdermal administration device of the above embodiment will be described. The third modified example differs from the first modified example in the arrangement of the adhesive sheet 40 and the configuration of the support body.

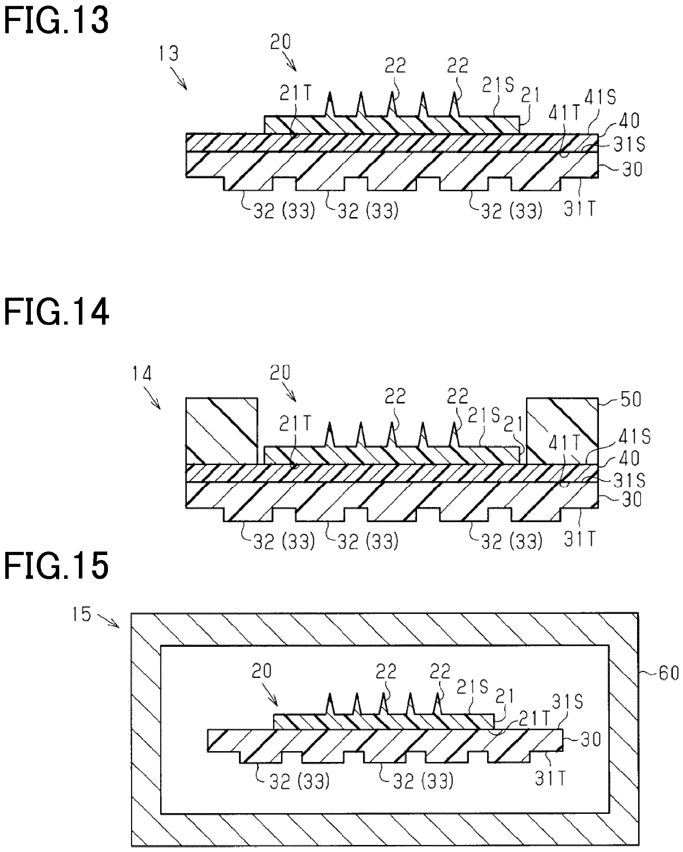

As shown in Fig. 13, in a transdermal administration device 13 of the third modified example, the adhesive sheet 40 is disposed between the microneedle 20 and the support substrate 30, and the support substrate 30 supports the adhesive sheet 40. The fixation surface 41T of the adhesive sheet 40 is fixed to the facing surface 31S of the support substrate 30, and the adhesive surface 41S is bonded to the second surface 21T of the substrate 21. The adhesive sheet 40 extends outward from the substrate 21 as seen in the direction perpendicular to the first surface 21S of the substrate 21 such that the adhesive surface 41S is exposed.

That is, in the third modified example, the adhesive sheet 40 is also located on a region which overlaps with the substrate 21 as viewed in the direction perpendicular to the first surface 21S of the substrate 21. In this configuration, the support body is composed of the support substrate 30 and the adhesive sheet 40, and the adhesive surface 41S configures the support surface of the support body.

According to this configuration, the adhesive sheet 40 has a simple shape compared with the first modified example and the second modified example having the adhesive sheet 40 formed in an annular shape which surrounds the substrate 21. Accordingly, the adhesive sheet 40 can be easily produced, and alignment in assembling the microneedle 20, the support substrate 30, and the adhesive sheet 40 can be easily performed.

Further, in the first and third modified examples, the adhesive surface 41S may not be necessarily disposed to surround the entire outer periphery of the substrate 21 as viewed in the direction perpendicular to the first surface 21S of the substrate 21, but may also be formed to extend outward from a portion of the outer periphery of the substrate 21.

### [Fourth Modified Example]

A fourth modified example of the transdermal administration device of the above embodiment will be described.

As shown in Fig. 14, a transdermal administration device 14 of the fourth modified example includes the microneedle 20, the support substrate 30, the adhesive sheet 40, and a protective sheet 50. The protective sheet 50 covers the adhesive surface 41S of the adhesive sheet 40 exposed outside the substrate 21 as seen in the direction perpendicular to the first surface 21S of the substrate 21.

The protective sheet 50 is attached to the adhesive surface 41S in a manner to be removable from the adhesive sheet 40 by a force applied by a user by holding and pulling the protective sheet 50. The protective sheet 50 is preferably made of a material formulated such that the adhesive surface 41S has adhesiveness that retains the adhesive sheet 40 to be adhered to the administration target for a desired period of time, and non-biocompatible material is not left on the adhesive surface 41S after the protective sheet 50 is peeled off from the adhesive sheet 40.

In use of the transdermal administration device 14, the protective sheet 50 is first peeled off from the adhesive sheet 40, and then the substrate 21 is bent along with the support substrate 30 and pressed against the skin of the administration target while the adhesive surface 41S of the adhesive sheet 40 is adhered to the skin of the administration target as with the first modified example.

According to this configuration, the adhesive surface 41S of the adhesive sheet 40 can be protected until it is adhered to the administration target.

In the thickness direction of the substrate 21, the end of the protective sheet 50 preferably protrudes from the tip of the projections 22. According to this configuration, the projections 22 can be prevented from being touched by a user's hand or other members. Accordingly, the projections 22 can also be protected by the protective sheet 50.

Although Fig. 14 shows an example of providing the protective sheet 50 in the configuration of the third modified example, the protective sheet 50 may also be provided in the configuration of the first modified example or the second modified example.

### [Fifth Modified Example]

A fifth modified example of the transdermal administration device of the above embodiment will be described.

As shown in Fig. 15, a transdermal administration device 15 of the fifth modified example includes the microneedle 20, the support substrate 30, and a case 60 that houses the microneedle 20 and the support substrate 30. The case 60 is made of a resin, for example, and has a box shape or a bag shape. The case 60 is configured such that the microneedle 20 and the support substrate 30 can be taken out from the case 60 by cutting off a portion of the case 60 or disassembling the case 60.

According to this configuration, the case 60 protects the microneedle 20, in particular, the projections 22. Further, the case 60 may not necessarily cover the entire microneedle 20 and the support substrate 30. For example, the case 60 may be formed as a lid that covers the microneedle 20 only.

### [Sixth Modified Example]

A sixth modified example of the transdermal administration device of the above embodiment will be described.

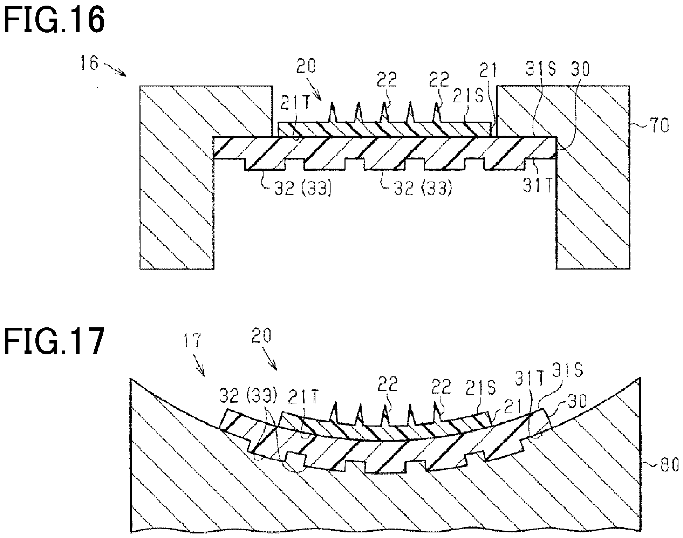

As shown in Fig. 16, a transdermal administration device 16 of the sixth modified example includes the microneedle 20, the support substrate 30, and an applicator 70 having a function of assisting puncturing of the skin by the projections 22. The applicator 70 may include a portion that serves as a grip for a user when the user pierces the projections 22 into the skin of the administration target, or a mechanism that applies a force on the support substrate 30 to bend the support substrate 30 and the substrate 21, or a mechanism that imparts a biasing force to the projections 22 for puncturing the skin.

The applicator 70 supports, for example, the outer periphery of the support substrate 30, and the projections 22 are exposed outside the applicator 70 from the end of the applicator 70.

### [Seventh Modified Example]

A seventh modified example of the transdermal administration device of the above embodiment will be described.

As shown in Fig. 17, a transdermal administration device 17 of the seventh modified example is used with a device having a mount 80 on which the support substrate 30 is mounted. This device may be, for example, a device for storing the transdermal administration device 17, a device for performing a predetermined process to the microneedle 20 before or after puncturing of the skin by the projections 22, or a device serving as a sensor that obtains information taking advantage of puncturing of the skin by the projections 22. The mount 80 has a reversed pattern of the raised and/or recessed portions of the raised and/or recessed surface 31T of the support substrate 30 such that the transdermal administration device 17 is mounted on the mount 80 when the raised and/or recessed portions of the raised and/or recessed surface 31T is fitted into the raised and/or recessed portions of the mount 80.

The transdermal administration device 17 may be mounted on the mount 80 while the substrate 21 is bent along with the support substrate 30. Alternatively, the support substrate 30 and the substrate 21 may be bent along with the mount 80 when the transdermal administration device 17 is used, or the support substrate 30 and the substrate 21 may be bent independently from the mount 80.

Further, the transdermal administration device according to the fifth to seventh modified examples may also include the adhesive sheet 40 or the protective sheet 50 as with the configuration described in the first to fourth modified examples.

### [Other Modified Example]

- The high rigidity region is a region having high rigidity in the support body and the low rigidity region is a region having low rigidity in the support body, and the support body may have any configuration having at least one of the high rigidity region and the low rigidity region extending in a strip shape or disposed in a strip shape. For example, in the configuration in which the support body is provided as a single member and the support body is made of a single material, a portion having an increased thickness in the support body is the high rigidity region and a portion having a reduced thickness in the support body is the low rigidity region. The thickness of the support body refers to the thickness of a space occupied by the material forming the support body. For example, in the configuration in which the support body is formed to have a flat surface and the support body includes pores inside thereof, a portion having low porosity in the support body is the high rigidity region and a portion having high porosity in the support body is the low rigidity region. Further, for example, in the configuration in which the material forming the support body includes a first material having high rigidity and a second material having rigidity lower than that of the first material, a portion made of the first material is the high rigidity region and a portion made of the second material is the low rigidity region. Further, for example, the support body may be formed as a laminate composed of a first support substrate made of the first material and a second support substrate made of the second material while the second support substrate is disposed across the entire facing surface 31S and the first support substrate formed in a strip shape is disposed on part of the second support substrate.
- In the above embodiments and the modified examples, the shape of the projection 22 in the administration section is not limited to a needle-shape, that is, a shape extending in the direction perpendicular to the first surface 21S of the substrate 21. The shape of the each of the projections 22 may be a blade shape, that is, a linear shape in which the projection 22 extends in one extending direction which is a direction extending along the first surface 21S of the substrate 21, and a distal end of the projection 22 extends in a direction which is not perpendicular to the first surface 21S of the substrate 21, for example, in a direction linearly extending along the extending direction. For example, the projection 22 may be formed as a triangular prism shape extending along the extending direction while one of three rectangular side surfaces of the triangular prism is in contact with the substrate 21 and the side of the triangular prism that partitions the other two side surfaces serves as a distal end of the projection 22.

As described above, according to the transdermal administration device of the present embodiment and the modified examples, the effects listed below can be achieved.
(1) When an external force is applied on the support substrate 30, the support substrate 30 is readily bent along with the substrate 21 at the bendable portion, which is the low rigidity region 35, and is not readily bent at the high rigidity region 34. As a result, when an external force is applied on the substrate 21, the shape of the substrate 21 is guided by the stripes 33 to become either a shape that is hardly bent at positions of the stripes 33 or a shape that is largely bent at positions of the stripes 33. Accordingly, variation in the shape of the substrate 21 can be reduced.

Further, the low rigidity region 35 and the high rigidity region 34 include the recessed element and the raised element, respectively, and the low rigidity region 35 and the high rigidity region 34 are defined by the thickness of the support substrate 30. That is, since the distribution of rigidity of the support substrate 30 can be determined by a level difference of the surface of the raised and/or recessed surface 31T, the high rigidity region 34 and the low rigidity region 35 can be easily defined in the support body.

(2) In the configuration in which the stripe 33 is formed of a single structure having a strip shape, which is the raised and/or recessed element 32, reproducibility of the shape of the substrate 21 which is guided by the stripes 33 can be improved. Further, in the configuration in which the stripes 33 are formed of a plurality of intermittent structures, which are the raised and/or recessed elements 32, a more complex shape of the substrate 21 which is guided by the stripes 33 may be easily contemplated.

(3) In the configuration having the plurality of stripes 33 formed on the raised and/or recessed surface 31T, the shape of the support substrate 30 can be readily guided by the stripes 33 compared with the configuration having a single stripe 33. Usually, a site of the skin of the administration target to which the transdermal administration device is applied rarely has a complex shape such as that formed of repetition of large raised and/or recessed shapes. In the configuration in which the plurality of stripes 33 do not intersect with each other, or in the configuration in which the plurality of stripes 33 are connected to each other at one of the ends of the stripes 33, the direction in which the support substrate 30 is readily bent is prevented from being complexly branched. Accordingly, these configurations are suitable for the application of the substrate 21 which is to be roughly bent, and the shape or position of the substrate 21 can be readily bent conforming to the shape of the skin.

(4) In the configuration in which the plurality of stripes 33 includes the stripes 33 that extend in a straight line, the shape guided by the stripes 33 can be simplified. In addition, for the stripes 33 extending in a straight line, positioning of the stripes 33 can be easily designed.

(5) Although a curved surface of the skin of the administration target to which the transdermal administration device is applied does not often have a complex shape in each administration site, the sizes or the curvatures are slightly different for each of the curved surfaces of the administration sites. In the configuration in which the plurality of stripes 33 include stripes 33 extending in different directions or stripes 33 extending in a curved line, a degree of curvature of the support substrate 30 can be varied in the support substrate 30. Accordingly, the substrate 21 can be further readily bent along the skin of the administration target by designing the arrangement or the shape or the stripes 33 appropriately for the degree of curvature of the administration site.

(6) In the configuration in which the entire substrate 21 overlaps with the support substrate 30 as viewed in the direction perpendicular to the first surface 21S, in particular, the entire substrate 21 overlaps with the region in which the stripes 33 are disposed, the entire second surface 21T is supported by the support substrate 30. Accordingly, the substrate 21 can be readily bent along with the support substrate 30 compared with a configuration in which part of the second surface 21T is supported by the support substrate 30. As a result, variation in the shape of the substrate 21 can be properly reduced, and thus the substrate 21 can be further readily bent along the skin of the administration target.

(7) In the configuration in which the transdermal administration device includes the adhesive sheet 40, the adhesive surface 41S can be adhered to the skin of the administration target to allow the substrate 21 to be easily bent along the skin of the administration target. Further, since the transdermal administration device can be fixed to the administration target while the substrate 21 is disposed along the skin of the administration target, the projections 22 can remain pierced into the administration target for a sufficient period of time.

### [Examples] (not claimed)

The aforementioned transdermal administration device will be described by using specific examples and comparative examples.

### <Example 1>

### (Fabrication of Transdermal Administration Device)

### Step 1. Fabrication of Intaglio Plate

First, an original plate for a microneedle was fabricated by micromachining. A silicon substrate was used for a material forming the original plate in which 60 projections were arrayed in a matrix of 6 × 10 at a pitch of 3 mm. Each of the projections was formed in a regular quadrangular pyramid (height: 120 µm, bottom: 38 µm × 38 µm). The 60 projections were disposed in a rectangular region having a size of approximately 30 mm × approximately 20 mm.

Then, the shape of the projections of the original plate was transferred by electroforming to thereby fabricate a nickel intaglio plate having a reversed pattern of raised and recessed portions of the original plate.

### Step 2. Preparation of Material Solution of Microneedle

Then, a material solution for the microneedle was prepared. Hydroxypropyl cellulose was dissolved in water using an acid, and the solution was then deaerated under a vacuum environment to prepare a material solution for the microneedle. The percentage of hydroxypropyl cellulose to the material solution was 5 wt%.

### Step 3. Forming of Microneedle

Then, the material solution for the microneedle prepared in Step 2 was supplied to the intaglio plate fabricated in Step 1 by an ink jet method so that the recessed portion is filled with the material solution. After the material solution in the recessed elements was dried, a molded product was removed from the intaglio plate to obtain a microneedle. The obtained microneedle had the substrate with 200 µm thickness and the projections with 120 µm length. In this microneedle, the outer shape of the substrate was formed into a rectangle of approximately 30 mm × approximately 20 mm.

### Step 4. Fabrication of Transdermal Administration Device

Then, a transdermal administration device was fabricated by assembling the microneedle formed in Step 3, a support substrate, and an adhesive sheet.

The support substrate was made of polyethylene, and an outer shape of the support substrate was a rectangle of approximately 40 mm × approximately 30 mm. On one of the surfaces of the support substrate, which was the raised and/or recessed surface, a plurality of raised elements extending in the same direction as the raised and/or recessed elements were disposed with an equal interval. The width of the raised and/or recessed elements in the direction perpendicular to the extending direction of the raised and/or recessed elements was 0.5 mm, and the interval between the adjacent raised and/or recessed elements was 0.5 mm. The maximum thickness TsM of the support substrate was 1 mm, and the minimum thickness Tsm was 0.5 mm, and the raised and/or recessed depth Ta was 0.5 mm.

The adhesive sheet was a skin patch adhesive sheet having adhesiveness on both surfaces, and the outer shape of the adhesive sheet was approximately 40 mm × approximately 30 mm. The thickness of the adhesive sheet was 500 µm.

A surface of the support substrate opposite to the raised and/or recessed surface on which the raised and/or recessed elements were formed, which was a facing surface, was bonded to one of the surfaces the adhesive sheet, a fixation surface. Then, the adhesive surface, which was a surface opposite to the fixation surface, was bonded to the second surface of the substrate so that the microneedles were positioned at the center of the adhesive surface.

Thus, the transdermal administration device of Example 1 was obtained. In the transdermal administration device of Example 1, the microneedles were positioned at the center of the adhesive sheet which was formed as a rectangle of approximately 40 mm × approximately 30 mm with the adhesive surface of 5 mm width was exposed surrounding the outer periphery of the substrate. Further, in the transdermal administration device, the raised and/or recessed surface having the stripes was located opposite to the projections with respect to the substrate.

### (Evaluation)

The transdermal administration device of Example 1 was placed on a curved site of the left upper arm with the extending direction of the stripes, that is, the extending direction of the raised and/or recessed elements being perpendicular to the curve direction of the skin. Then, the transdermal administration device was pressed against the skin by applying a force on the support substrate by fingers. As a result, the substrate was easily bent along the skin. Since the substrate made of hydroxypropyl cellulose had low rigidity, which makes difficult to control deformation, the substrate was difficult to bend into the same shape every time of usage if used alone. However, when the substrate was bent along with the support substrate, the substrate can be easily bent conforming to the support substrate. In addition, reproducibility of the bending shape was observed. Further, the transdermal administration device was fixed to the skin with the substrate bent along the skin by virtue of the adhesive sheet adhered to the skin.

Moreover, when picking up the transdermal administration device by fingers, handling of the transdermal administration device was easy since the raised and/or recessed portions on the raised and/or recessed surface of the support substrate were caught by fingers for slippage prevention.

### <Comparative Example 1>

### (Fabrication of Transdermal Administration Device)

The transdermal administration device of Comparative Example 1 was fabricated by the same steps as those of Example 1 except for the configuration of the support substrate.

The support substrate was made of polyethylene, and an outer shape of the support substrate was a rectangle of approximately 40 mm × approximately 30 mm. The support substrate had a plate shape, did not include the raised and/or recessed elements, and had a constant thickness of 1 mm.

### (Evaluation)

The transdermal administration device of Comparative Example 1 was pressed against a curved site of the left upper arm. However, since the support substrate had high rigidity, the support substrate was difficult to bend. Although it was attempted to bend the substrate along with the support substrate by applying a strong force, the direction in which the support substrate and the substrate were bent varied each time a force was applied. As a result, it was difficult to bend the substrate into a specific shape along the skin. Although the transdermal administration device was pressed against the skin by applying a force on the support substrate by fingers, the ends of the support substrate and the substrate which was bent along with the support substrate were detached from the skin. As a result, the substrate failed to be bent along the skin.

### <Comparative Example 2>

### (Fabrication of Transdermal Administration Device)

The transdermal administration device of Comparative Example 2 was fabricated by the same steps as those of Example 1 except for the configuration of the support substrate.

The support substrate was made of polyethylene, and an outer shape of the support substrate was a rectangle of approximately 40 mm × approximately 30 mm. The support substrate had a plate shape, did not include the raised and/or recessed elements, and had a constant thickness of 0.5 mm.

### (Evaluation)

The transdermal administration device of Comparative Example 2 was pressed against a curved site of the left upper arm. However, since the support substrate had low rigidity and the support substrate was subject to deformation, it was difficult to bend the substrate into a specific direction, and the direction in which the support substrate was bent varied each time a force was applied. As a result, it was difficult to bend the substrate, which deformed conforming to the support substrate, into a specific shape along the skin. That is, control of deformation of the support substrate and the substrate was difficult, and the substrate was wrinkled, which causes air bubbles to enter between the substrate and the skin. As a result, the substrate failed to be bent along the skin.

### <Example 2>

### (Fabrication of Transdermal Administration Device)

The transdermal administration device of Example 2 was fabricated by the same steps as those of Example 1 except that chitosan instead of hydroxypropyl cellulose was used for forming the microneedle. The percentage of chitosan to the material solution of the microneedle was 5 wt%.

### (Evaluation)

The transdermal administration device of Example 2 was placed on a curved site of the left upper arm with the extending direction of the stripes, that is, the extending direction of the raised and/or recessed elements being perpendicular to the curve direction of the skin. Then, the transdermal administration device was pressed against the skin by applying a force on the support substrate by fingers. As a result, the substrate was easily bent along the skin. Since the substrate made of chitosan had high rigidity and caused high resistance during bending, the substrate was difficult to be bent into the same shape every time of usage if used alone. However, when the substrate was bent along with the support substrate, the substrate can be easily bent, and reproducibility of the bending shape was observed. Moreover, the transdermal administration device was fixed to the skin with the substrate bent along the skin by virtue of the adhesive sheet adhered to the skin.

Moreover, when picking the transdermal administration device by fingers, handling of the transdermal administration device was easy since the raised and/or recessed portions on the raised and/or recessed surface of the support substrate were caught by fingers for slippage prevention.

### <Comparative Example 3>

### (Fabrication of Transdermal Administration Device)

The transdermal administration device of Comparative Example 3 was fabricated by the same steps as those of Example 2 except for the configuration of the support substrate.

The support substrate was made of polyethylene, and an outer shape of the support substrate was a rectangle of approximately 40 mm × approximately 30 mm. The support substrate had a plate shape, did not include the raised and/or recessed elements, and had a constant thickness of 1 mm.

### (Evaluation)

The transdermal administration device of Comparative Example 3 was pressed against a curved site of the left upper arm. However, since the support substrate had high rigidity, the support substrate was difficult to be bent, and bending of the substrate along with the support substrate was difficult. Although it was attempted to bend the substrate along with the support substrate by applying a strong force, the direction in which the support substrate and the substrate were bent varied each time a force was applied. As a result, it was difficult to bend the substrate into a specific shape along the skin. Although the transdermal administration device was pressed against the skin by applying a force on the support substrate by fingers, the ends of the support substrate and the substrate were detached from the skin. As a result, the substrate failed to be bent along the skin.

### <Comparative Example 4>

### (Fabrication of Transdermal Administration Device)

The transdermal administration device of Comparative Example 4 was fabricated by the same steps as those of Example 2 except for the configuration of the support substrate.

The support substrate was made of polyethylene, and an outer shape of the support substrate was a rectangle of approximately 40 mm × approximately 30 mm. The support substrate had a plate shape, did not include the raised and/or recessed elements, and had a constant thickness of 0.02 mm.

### (Evaluation)

The transdermal administration device of Comparative Example 4 was pressed against a curved site of the left upper arm. However, since the support substrate had low rigidity and the support substrate was subject to deformation, it was difficult to bend the substrate into a specific direction, and the direction in which the support substrate was bent varied each time a force was applied. As a result, it was difficult to apply a force on the substrate so as to bend it into a specific direction via the support substrate, and thus difficult to bend the substrate into a specific shape along the skin. Although the transdermal administration device was pressed against the skin by applying a force on the support substrate by fingers, the end of the substrate was detached from the skin. As a result, the substrate was failed to be bent along the skin.

As described above, it was confirmed that the transdermal administration device which included the support substrate having the raised and/or recessed surface on which the stripes were formed could reduce variation in the shape of the substrate when an external force was applied on the substrate regardless of whether the substrate had high or low rigidity, and the substrate could be easily bent along the administration target.

### [Reference Signs List]

10 to 17: Transdermal administration device, 20: Microneedle, 21: Substrate, 21S: First surface, 21T: Second surface, 22: Projection, 30: Support substrate, 31S: Facing surface, 31T: Raised and/or recessed surface, 32: Raised and/or recessed element, 33: Stripe, 34: High rigidity region, 35: Low rigidity region, 40: Adhesive sheet, 41S: Adhesive surface, 41T: Fixation surface, 50: Protective sheet, 60: Case, 70: Applicator, 80: Mount

## Claims

1. A transdermal administration device (10 to 17) comprising:
an administration section (20) which includes a substrate (21) having a first surface (21S) and a second surface (21T) which is a surface opposite to the first surface (21S), and a projection (22) protruding from the first surface (21S), the administration section (20) having flexibility; **characterized by**
a support body (30) having a plate shape and a support surface (31S) that supports the second surface (21T), the support body (30) having flexibility, wherein the support surface (31S) is a flat surface and is bonded to the second surface (21T) by adhesiveness of an adhesive applied, wherein
the support body (30) includes at least one low rigidity region (35), and at least one high rigidity region (34) having flexural rigidity higher than flexural rigidity of the low rigidity region (35), the support body (30) being configured to satisfy at least one of requirements that the low rigidity region (35) is sandwiched by two high rigidity regions (34) to form a stripe and that the high rigidity region (34) is sandwiched by two low rigidity regions (35) to form a stripe (33), wherein
the support body (30) has a raised and/or recessed surface (31T) which is a surface opposite to the support surface (31S),
the high rigidity region (34) includes a raised element raised from the raised and/or recessed surface (31T), and
the low rigidity region (35) includes a recessed element recessed from the raised and/or recessed surface (31T).

2. The transdermal administration device according to claim 1, wherein the stripe (33) is composed of a single structure that extends in a strip shape.

3. The transdermal administration device according to claim 1, wherein the stripe (33) is composed of a plurality of intermittent structures (32).

4. The transdermal administration device according to any one of claims 1 to 3, comprising a plurality of the stripes (33), wherein the plurality of stripes (33) includes the stripes (33) that do not intersect with each other.

5. The transdermal administration device according to any one of claims 1 to 4, comprising a plurality of stripes (33) having an end and disposed adjacent to each other, the plurality of stripes (33) including stripes (33) that are connected to each other at the ends.

6. The transdermal administration device according to any one of claims 1 to 5, comprising a plurality of the stripes (33), wherein the plurality of stripes (33) includes the stripes (33) that extend in different directions from each other.

7. The transdermal administration device according to any one of claims 1 to 6, comprising a plurality of the stripes (33), wherein the plurality of stripes (33) includes at least one stripe (33) that extends in a straight line.

8. The transdermal administration device according to any one of claims 1 to 7, comprising a plurality of the stripes (33), wherein the plurality of stripes (33) includes at least one stripe (33) that extends in a curved line.

9. The transdermal administration device according to any one of claims 1 to 8, comprising an adhesive sheet (40) having an adhesive surface (41S), wherein the adhesive surface (41S) is located outside the substrate (21) as viewed in a direction perpendicular to the first surface (21S).

## Patentansprüche

1. Transdermale Verabreichungsvorrichtung (10 bis 17) mit:
einem Verabreichungsabschnitt (20), welcher ein Substrat (21) mit einer ersten Fläche (21S) und einer zweiten Fläche (21T), welche eine zu der ersten Fläche (21S) entgegengesetzten Fläche ist, und einen Vorsprung (22) hat, der von der ersten Fläche (21S) vorsteht, wobei der Verabreichungsabschnitt (20) Flexibilität aufweist; **gekennzeichnet durch**
einen Stützkörper (30) mit einer Plattenform und einer Stützfläche (31S), die die zweite Fläche (21T) stützt, wobei der Stützkörper (30) Flexibilität aufweist, wobei die Stützfläche (31S) eine ebene Fläche ist und mit der zweiten Fläche (21T) durch das Klebevermögen eines aufgebrachten Klebstoffs gefügt ist, wobei
der Stützkörper (30) mindestens einen Bereich (35) mit geringer Steifigkeit und mindestens einen Bereich (34) mit hoher Steifigkeit hat, der eine höhere Biegesteifigkeit als eine Biegesteifigkeit des Bereichs (35) mit geringer Steifigkeit aufweist, wobei der Stützkörper (30) konfiguriert ist, mindestens eine der Anforderungen zu erfüllen, dass der Bereich (35) mit geringer Steifigkeit von zwei Bereichen (34) mit hoher Steifigkeit eingeschlossen ist, um einen Streifen auszubilden, und dass der Bereich (34) mit hoher Steifigkeit von zwei Bereichen (35) mit geringer Steifigkeit eingeschlossen ist, um einen Streifen (33) auszubilden, wobei
der Stützkörper (30) eine erhöhte und/oder vertiefte Fläche (31T) hat, welche eine Fläche gegenüber der Stützfläche (31S) ist,
der Bereich (34) mit hoher Steifigkeit ein erhöhtes Element hat, das sich von der erhöhten und/oder vertieften Fläche (31T) erhöht ist, und
der Bereich (35) mit geringer Steifigkeit ein vertieftes Element hat, das von der erhöhten und/oder vertieften Fläche (31T) vertieft ist.

2. Transdermale Verabreichungsvorrichtung nach Anspruch 1, wobei der Streifen (33) aus einer einzelnen Struktur gebildet ist, die sich in einer Streifenform erstreckt.

3. Transdermale Verabreichungsvorrichtung nach Anspruch 1, wobei der Streifen (33) aus einer Vielzahl von unterbrochenen Strukturen (32) gebildet ist.

4. Transdermale Verabreichungsvorrichtung nach einem der Ansprüche 1 bis 3 mit einer Vielzahl der Streifen (33), wobei die Vielzahl von Streifen (33) die Streifen (33) aufweist, die einander nicht kreuzen.

5. Transdermale Verabreichungsvorrichtung nach einem der Ansprüche 1 bis 4 mit einer Vielzahl von Streifen (33), die ein Ende haben und benachbart zueinander angeordnet sind, wobei die Vielzahl von Streifen (33) Streifen (33) hat, die an den Enden miteinander verbunden sind.

6. Transdermale Verabreichungsvorrichtung nach einem der Ansprüche 1 bis 5 mit einer Vielzahl der Streifen (33), wobei die Vielzahl von Streifen (33) die Streifen (33) hat, die sich in unterschiedliche Richtungen voneinander erstrecken.

7. Transdermale Verabreichungsvorrichtung nach einem der Ansprüche 1 bis 6 mit einer Vielzahl der Streifen (33), wobei die Vielzahl von Streifen (33) mindestens einen Streifen (33) hat, der sich in einer geraden Linie erstreckt.

8. Transdermale Verabreichungsvorrichtung nach einem der Ansprüche 1 bis 7 mit einer Vielzahl der Streifen (33), wobei die Vielzahl von Streifen (33) mindestens einen Streifen (33) hat, der sich in einer gekrümmten Linie erstreckt.

9. Transdermale Verabreichungsvorrichtung nach einem der Ansprüche 1 bis 8 mit einem klebenden Blatt (40), das eine klebende Fläche (41S) hat, wobei die klebende Fläche (41S) bei Betrachtung in einer Richtung senkrecht zur ersten Fläche (21S) außerhalb des Substrats (21) gelegen ist.

## Revendications

1. Dispositif d'administration transdermique (10 à 17) comprenant :
une section d'administration (20) qui comporte un substrat (21) ayant une première surface (21S) et une deuxième surface (21T) qui est une surface à l'opposé de la première surface (21S), et une saillie (22) faisant saillie depuis la première surface (21S), la section d'administration (20) ayant une flexibilité ; **caractérisé par**
un corps de support (30) ayant une forme de plaque et une surface de support (31S) qui supporte la deuxième surface (21T), le corps de support (30) ayant une flexibilité, dans lequel la surface de support (31S) est une surface plate et est collée à la deuxième surface (21T) pas adhésivité d'un adhésif appliqué, dans lequel
le corps de support (30) comporte au moins une région de faible rigidité (35), et au moins une région de forte rigidité (34) ayant une rigidité en flexion supérieure à la rigidité en flexion de la région de faible rigidité (35), le corps de support (30) étant configuré pour satisfaire au moins une des exigences selon lesquelles la région de faible rigidité (35) est prise en sandwich par deux régions de forte rigidité (34) pour former une bande et la région de forte rigidité (34) est prise en sandwich par deux régions de faible rigidité (35) pour former une bande (33), dans lequel le corps de support (30) a une surface surélevée et/ou renfoncée (31T) qui est une surface à l'opposé de la surface de support (31S),
la région de forte rigidité (34) comporte un élément surélevé, surélevé depuis la surface surélevée et/ou renfoncée (31T), et
la région de faible rigidité (35) comporte un élément renfoncé, renfoncé depuis la surface surélevée et/ou renfoncée (31T).

2. Dispositif d'administration transdermique selon la revendication 1, dans lequel la bande (33) est composée d'une seule structure qui s'étend en forme de bande.

3. Dispositif d'administration transdermique selon la revendication 1, dans lequel la bande (33) est composée d'une pluralité de structures intermittentes (32).

4. Dispositif d'administration transdermique selon l'une quelconque des revendications 1 à 3, comprenant une pluralité des bandes (33), dans lequel la pluralité de bandes (33) comporte les bandes (33) qui ne se croisent pas.

5. Dispositif d'administration transdermique selon l'une quelconque des revendications 1 à 4, comprenant une pluralité de bandes (33) ayant une extrémité et disposées au voisinage les unes des autres, la pluralité de bandes (33) comportant des bandes (33) qui sont reliées les unes aux autres aux extrémités.

6. Dispositif d'administration transdermique selon l'une quelconque des revendications 1 à 5, comprenant une pluralité des bandes (33), dans lequel la pluralité de bandes (33) comporte les bandes (33) qui s'étendent dans différentes directions les unes des autres.

7. Dispositif d'administration transdermique selon l'une quelconque des revendications 1 à 6, comprenant une pluralité des bandes (33), dans lequel la pluralité de bandes (33) comporte au moins une bande (33) qui s'étend en ligne droite.

8. Dispositif d'administration transdermique selon l'une quelconque des revendications 1 à 7, comprenant une pluralité des bandes (33), dans lequel la pluralité de bandes (33) comporte au moins une bande (33) qui s'étend en ligne courbe.

9. Dispositif d'administration transdermique selon l'une quelconque des revendications 1 à 8, comprenant une feuille adhésive (40) ayant une surface adhésive (41S), dans lequel la surface adhésive (41S) est située à l'extérieur du substrat (21), observée dans une direction perpendiculaire à la première surface (21S).
